(19) Europäisches Patentamt

European Patent Office

Office européen des brevets

(11) Veröffentlichungsnummer: **0 009 596**
**B1**

(12) **EUROPÄISCHE PATENTSCHRIFT**

(45) Veröffentlichungstag der Patentschrift: **15.12.82**

(21) Anmeldenummer: **79103070.3**

(22) Anmeldetag: **21.08.79**

(51) Int. Cl.³: **G 01 N 33/96,**
**G 01 N 33/92**

(54) **Wässrige Lipid-Standardlösung.**

(30) Priorität: **11.09.78 DE 2839433**

(43) Veröffentlichungstag der Anmeldung:
**16.04.80 Patentblatt 80/8**

(45) Bekanntmachung des Hinweises auf die Patenterteilung:
**15.12.82 Patentblatt 82/50**

(84) Benannte Vertragsstaaten:
**BE CH DE FR GB IT NL SE**

(56) Entgegenhaltungen:
**DE - A - 2 653 537**
**DE - B - 1 234 417**
**DE - B - 1 523 133**
**DE - B - 2 324 386**
**US - A - 2 863 734**
**US - A - 3 859 047**
**US - A - 4 110 077**

(73) Patentinhaber: **Merck Patent Gesellschaft mit beschränkter Haftung**
**Frankfurter Strasse 250**
**D-6100 Darmstadt (DE)**

(72) Erfinder: **Harders, Hans-Dieter, Dr.**
**Holzhofallee 1**
**D-6100 Darmstadt (DE)**
Erfinder: **Helger, Roland, Dr.**
**Ludwigshöhstrasse 85**
**D-6100 Darmstadt (DE)**

Courier Press, Leamington Spa, England.

## 0 009 596

### Wäßrige Lipid-Standardlösung

Die Erfindung betrifft eine wäßrige Lipid-Standardlösung, die neben einem Lipid ein ionisches und ein nichtionisches Detergens enthält.

Für klinisch-chemische Messungen werden in der Regel Standardlösungen benötigt, deren Eigenschaften möglichst denen eines Serums ähneln sollen. Dazu muß die betreffende Substanz in einem wäßrigen System gelöst werden, da sich nur so die Standardprobe wie ein Serum verhält. Eine solche Standardlösung sollte außerdem ausreichend stabil sein und sich über einen längeren Zeitraum hinweg nicht verändern. Diese Forderungen sind besonders schwer zu erfüllen bei Standardlösungen für die Bestimmung von Lipiden, z.B. von Cholesterin oder von Triglyceriden. Für Cholesterin sind in der Literatur außer der Verwendung von organischen Lösungsmitteln folgende wäßrige Lösungsmittelsysteme beschrieben: Nichtionische Detergentien (DE—B—15 23 133, Ärztl. Lab. *20*, 148—151 (1974)) und Wasser-Alkohol-Mischungen mit einem Zusatz an Detergentien (DE—B—23 24 386). Aus der DE—A—26 53 537 ist u. a. ein Verfahren zur Bestimmung von Cholesterin bekannt, wobei die dort verwendete Reagenslösung nichtionische und ionische Detergentien enthält. Diese Lösung enthält jedoch kein Cholesterin und die Detergentienkonzentration liegt um Größenordnungen unterhalb der für eine stabile Standardlösung notwendigen Konzentration. In der DE—B—12 34 417 ist ein Testreagens zur Bestimmung des Fettgehalts beschrieben, das Detergentien und Alkohol in wäßriger Lösung enthält. Auch dieses Reagens enthält keine Lipide, es besteht zum größten Teil aus Detergentien und Alkohol und ist allein aufgrund des Alkoholgehalts als Standardlösung völlig ungeeignet. Die aus dem Stand der Technik bekannten Standardlösungen, die mit Detergentien hergestellt werden, sind vor allem bei Cholesterinkonzentrationen im pathologischen Bereich nur wenige Wochen stabil; Standardlösungen mit Alkoholzusatz verdunsten wegen ihres Gehaltes an Alkoholen wesentlich stärker als eine Serumprobe, selbst bei Verwendung höher siedender Alkohole. Eine Senkung des Alkoholgehaltes führt zur Ausfällung des Lipids; die gleichzeitige Erhöhung der Detergenskonzentration resultiert in einer unerwünscht hohen Viskositat.

Der Erfindung liegt die Aufgabe zugrunde, eine alkoholfreie, stabile, wäßrige Lipid-Standardlösung zur Verfügung zu stellen, die einem Serum möglichst nahe kommt, beim unvermeidlichen Stehen der Proben möglichst wenig verdunstet und deren Viskosität die Dosierung nicht beeinflußt.

Erfindungsgemäß wurde die Aufgabe durch eine wäßrige, alkoholfreie Lipid-Standardlösung gelöst, die neben einem Lipid ein nichtionisches und ein ionisches Detergens enthält.

Gegenstand der Erfindung sind wäßrige Lipid-Standardlösungen, enthaltend mindestens ein Lipid und ein nichtionisches Detergens, die gekennzeichnet sind durch einen Gehalt von 0,5—10 Gew.-% eines ionischen Detergens.

Überraschenderweise hat sich gezeigt, daß es möglich ist, eine alkoholfreie, stabile, wäßrige Standardlösung herzustellen, wenn man zusätzlich zu einem der in den oben angegebenen Publikationen genannten nichtionischen Detergentien ein ionisches Detergens in der relativ hohen Konzentration von 0,5—10 Gew.-% verwendet. Das Lösungsvermögen dieser Mischungen ist dann sogar so gut, daß auch Cholesterinester langkettiger Fettsäuren, die die überwiegende Form des Cholesterins im Serum sind, in Lösung gehalten werden können, obwohl diese wesentlich unpolarer und schlechter in Wasser löslich sind als freies Cholesterin. Auch ist es damit möglich, andere Lipide, deren Bestimmung klinische Bedeutung haben, wie Triglyceride oder Lecithin, in diese Lösung einzubringen.

Die erfindungsgemäße wäßrige Lipid-Standardlösung enthält neben 0,2—4 g/l Cholesterin und/oder 0,1—2 g/l Triolein etwa 2—20 Gew.-%, vorzugsweise 5—15 Gew.-%, nichtionisches Detergens und einen zusätzlichen Gehalt von etwa 0,5—10 Gew.-% vorzugsweise 1—5 Gew.-% ionisches Detergens.

Als nichtionische Detergentien kommen z.B. Polyäthylenglykolmonoalkylphenyläther, Diäthylen-glykolmonobutyläther, Oxypolyäthoxydodecan, Octylphenoxypolyäthoxyäthanol, Polyäthoxyäthylen-sorbitanfettsäureester in Frage, vorzugsweise Polyäthylenglykolmonoalkylphenyläther.

Geeignete ionische Detergentien sind kationische Detergentien wie Alkylbenzyldimethylammoniumchlorid, Alkylbenzyltrimethylammoniumchlorid, Methylbenzethoniumchlorid. Geeignet sind weiterhin anionische Detergentien wie Natriumdioctylsulfosuccinat, Natriumtriisopropylnaphthalinsulfonat, Natriumdodecylsulfat, Natrium-N-lauroyl-sarkosinat.

Die Stardardlösung kann zusätzlich weitere Lipide enthalten, die klinisch bedeutsam sind, wie Cholesterinester langkettiger Fettsäuren (0,1—2 g/l) und/oder Lecithin (0,2—4 g/l). Gegebenenfalls kann der pH-Wert der Standardlösung im Bereich von 6—8 mit Hilfe eines Puffers eingestellt werden. Als geeignete Puffer haben sich z.B. Tris-, Phosphat-, Triäthanolamin- oder Imidazol-Puffer in einer Konzentration von 10—100 mmol/l erwiesen, bevorzugt wird ein Tris-HCl-Puffer vom pH-Wert 7,4 in einer Konzentration von 20 mmol/l verwendet. Zur Vermeidung mikrobieller Kontamination können noch 0,5—20 g/l eines Alkaliazids zugesetzt werden, vorzugsweise 1 g/l Natriumazid.

Die Herstellung bekannter Lipid-Standardlösungen oder -Emulsionen erfolgt durch Ultraschallbehandlung oder durch vorsichtiges und langsames Verdünnen einer Mischung aus Cholesterin und Detergens mit Wasser. Ersteres erfordert aufwendige technische Apparaturen und arbeitstechnische Sicherheitsmaßnahmen, letzteres eine erhebliche Sorgfalt beim Zufügen des Wassers, um eine Ausfällung des Cholesterins zu vermeiden. Diese Nachteile lassen sich umgehen, wenn man die neue Lipid-Stanardlösung in der Weise herstellt, daß man die Lipide zusammen mit den Detergentien in einem leicht flüchtigen organischen Lösungsmittel, z.B. Dichlormethan oder Methanol, löst und das Lösungsmittel unter vermindertem Druck abdampft. Dadurch wird eine gute Durchmischung der Lipide und der Detergentien erreicht. Anschließend wird die benötigte Menge Wasser, gegebenenfalls unter leichtem Erwärmen, hinzugegeben. Das Wasser kann zusätzlich eine Alkaliazid und einen Puffer enthalten.

Die Erfindung wird durch die folgenden Beispiele näher erläutert.

### Beispiel 1

In 25 ml Dichlormethan werden

300 mg Cholesterin,
200 mg Cholesterinliholat,
200 mg Triolein,
100 mg Lecithin,
5 g  Polyäthylenglykol-mono-[p-(1,1,3,3-tetramethylbutyl)-phenyl]-äther und
1 g  Natriumdioctylsulfosuccinat

gelöst. Das Dichlormethan wird unter vermindertem Druck abgedampft und der Rückstand mit 80 ml Wasser von ca 40°C aufgenommen; nach dem Erkalten wird mit Wasser auf 100 ml aufgefüllt. Man erhält eine klare, stabile Lipid-Standardlösung.

### Beispiel 2

In 25 ml Dichlormethan werden
300 mg Cholesterin,
5 g  Polyäthylenglykolmonooctylphenyläther und
1 g  Natriumdodecylsulfat

gelöst. Das Dichlormethan wird unter vermindertem Druck abgedampft und der Rückstand wie in Beispiel 1 beschrieben mit Wasser aufgenommen und aufgefüllt.

### Beispiel 3

In 25 ml Methanol werden
300 mg Cholesterin,
200 mg Cholesterinlinolat,
200 mg Triolein,
10 g  Polyäthylenglykolmonooctylphenyläther und
5 g  Alkylbenzyldimethylammoniumchlorid

gelöst. Der Alkohol wird unter vermindertem Druck abgedampft und der Rückstand wie in Beispiel 1 beschrieben mit Wasser aufgenommen und aufgefüllt.

### Beispiel 4

In 25 ml Methanol werden
300 mg Cholesterin,
5 g  Polyäthylenglykolmonooctylphenyläther und
2 g  Methylbenzethoniumchlorid

gelöst. Der Alkohol wird unter vermindertem Druck abgedampft und der Rückstand wird mit warmen Tris-HCl-Puffer (20 mmol/l; pH 7,4), der zusätzlich 1 g/l Natriumazid enthält, aufgenommen und nach dem Erkalten mit dieser Lösung auf 100 ml aufgefüllt.

**Patentansprüche**

1. Wäßrige Lipid-Standardlösung, enthaltend mindestens ein Lipid und ein nichtionisches Detergens, gekennzeichnet durch einen Gehalt von 0,5 bis 10 Gew.-% eines ionischen Detergens.
2. Lipid-Standardlösung nach Anspruch 1, gekennzeichnet durch einen zusätzlichen Gehalt an Puffer und Alkali-azid.

**Claims**

1. Aqueous lipid standard solution, containing at least one lipid and a non-ionic detergent, characterised in that it contains from 0.5 to 10% by weight of an ionic degergent.
2. Lipid standard solution according to Claim 1, characterised in that it additionally contains a buffer and an alkali metal azide.

# 0 009 596

**Revendications**

1. Solution étalon aqueuse de lipide contenant au moins un lipide et un détergent non-ionique et ɔractérisée en ce qu'elle contient de 0,5 à 10% en poids d'un détergent ionique.

2. Solution étalon de lipide selon la revendication 1, caractérisée en ce qu'elle contient en outre un tampon et un azide alcalin.